# EUROPEAN PATENT SPECIFICATION

(11) **EP 3 230 783 B1**
(45) Date of publication and mention of the grant of the patent: **27.01.2021**
(21) Application number: 15817040.7
(22) Date of filing: 10.12.2015
(51) Int. Cl.: A61N 5/06, G02B 27/09, G02B 19/00

(54) **HANDHELD, LOW-LEVEL LASER APPARATUSES AND METHODS FOR LOW-LEVEL LASER BEAM PRODUCTION**
TRAGBARE LOW-LEVEL-LASERVORRICHTUNG UND VERFAHREN ZUR LOW-LEVEL-LASERSTRAHLERZEUGUNG
APPAREILS LASER À BAS NIVEAU PORTATIFS ET PROCÉDÉS DE PRODUCTION DE FAISCEAU LASER À BAS NIVEAU

(30) Priority: 10.12.2014 US 201462090307 P; 09.12.2015 US 201514963511
(43) Date of publication of application: 18.10.2017
(73) Proprietor: Medical Coherence LLC, Fairfield, NJ 07004 (US)
(72) Inventor: GERLITZ, Yonatan, Herzliya (IL)
(74) Representative: Ward, David Ian
(86) International application number: PCT/US2015/065079
(87) International publication number: WO 2016/094712

(56) References cited:
- US-A1- 2008 077 198
- US-A1- 2013 317 571

## Description

### BACKGROUND

The present disclosure relates generally to the field of laser beam production, and more particularly to systems and methods for low-level laser beam production. Known low-level lasers produce a beam originating from the diode with an initial irradiance of about 5 mW/cm² (milliWatts/centimeter²) to about 5 W/cm², such as 5 mW/cm² to about 1 W/cm². "Irradiance" refers to the power, or energy per time, per unit area of a laser emission. Irradiance might also be called "power density." Laser beams tend to have narrow cross sections. However, in some applications, particularly in the medical field, it may be beneficial to apply a laser beam over large areas.

Raw laser beams may lack coherence and/or may be too narrow to be effectively applied to a large area. Further, raw laser emissions may have high peak irradiance that may injure a person if directed into sensitive tissue such as an eye. As an additional drawback, particularly with respect to infrared (IR) laser beams, which are not humanly visible, it may be difficult to determine where the laser is pointing, increasing the risk of accidentally directing the laser into sensitive tissue. Known handheld laser apparatuses are disclosed in US2013/0317571 A1 and US2008/0077198 A1.

### SUMMARY

The invention defines a handheld low-level laser apparatus according to claim 1 and a method of producing a low-level laser beam according to claim 12. Methods and apparatuses are described herein that may overcome one or more of the deficiencies described above. One handheld, low-level laser apparatus includes a laser diode configured to generate an infrared (IR) laser emission propagating along a laser path. One or more corrective lens is positioned in the laser path and is configured to receive the IR laser emission therethrough. The one or more corrective lens is further configured alone or in combination to correct the astigmatism of the IR laser emission and to collimate the IR laser emission. A divergence lens is positioned in the laser path after the one or more corrective lens and is configured to receive the IR laser emission therethrough and to diverge the IR laser emission after the IR laser emission passes through the one or more corrective lens. A front lens is positioned in the laser path after the divergence lens and is configured to receive the IR laser emission therethrough and to collimate the IR laser emission after the IR laser emission passes through the divergence lens.

One low-level laser beam producing method includes, using a laser diode, generating an IR laser emission having an astigmatism and propagating the IR laser emission along a laser path. One or more corrective lens positioned in the laser path are used to receive the IR laser emission therethrough and, using the one or more corrective lens alone or in combination, to correct the astigmatism of the IR laser emission and collimate the IR laser emission. The method includes using a divergence lens positioned in the laser path after the one or more corrective lens, receiving the IR laser emission therethrough and diverging the IR laser emission after the IR laser emission passes through the one or more corrective lens. A front lens positioned in the laser path after the divergence lens receives the IR laser emission therethrough and collimates the IR laser emission after the IR laser emission passes through the divergence lens.

Another low-level laser beam producing method includes repeatedly directing an IR laser emission through a series of lenses during a first set of time periods. The method further includes repeatedly directing a visible laser emission through the series of lenses during a second set of time periods, each time period of the first set of time periods being distinct from each time period of the second set of time periods. In the method, the series of lenses increases collimation, which increases coherence, of a received laser emission corresponding to the IR laser emission, the visible laser emission, or both by collimating the received laser emission and correcting an astigmatism of the received laser emission, thereby forming a corrected laser emission. The series of lenses enlarges a cross section of the corrected laser emission, thereby forming an enlarged laser emission. The series of lenses also collimates the enlarged laser emission.

The features, functions, and benefits that have been discussed can be achieved independently in various embodiments or may be combined in yet other embodiments further details of which can be seen with reference to the following description and drawings.

### BRIEF DESCRIPTION OF THE DRAWINGS

Some embodiments are described below with reference to the following accompanying drawings.
FIG. 1A depicts a low-level laser apparatus;
FIG. 1B depicts a cross-section of a raw laser emission;
FIG. 1C depicts a cross-section of the corrected laser emission 152;
FIG. 1D depicts a cross-section of the high-coherence laser emission 156;
FIG. 2 depicts another low-level laser apparatus;
FIG. 3 depicts a timing diagram illustrating a time-sharing duty cycle for operating a first laser diode and a second laser diode;
FIG. 4 depicts applying a low-level laser with increased coherence to an area;
FIGS. 5A-5C depict a low-level laser apparatus with interchangeable front lens assemblies;
FIGS. 6-9 are irradiance distribution graphs in units of W/cm² showing optical performance for different levels of laser beam expansion;
FIG. 10 is a flow chart of a low-level laser beam producing method; and
FIG. 11 is a flow chart of another low-level laser beam producing method.

### DETAILED DESCRIPTION

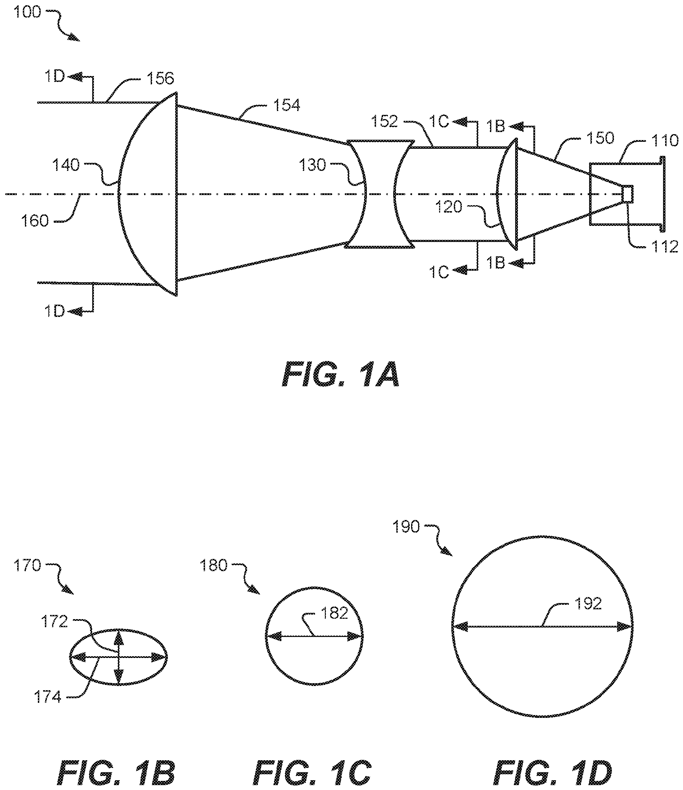

Referring to FIG. 1A, a low-level laser apparatus is depicted and generally designated 100. The apparatus 100 includes a laser source 110, a corrective lens 120, a divergence lens 130, and a front lens 140. Together, the corrective lens 120, the divergence lens 130, and the front lens 140 make up a series of lenses of an optical system that collimates in two stages and expands a cross-section of a laser beam between stages, thereby increasing the coherence of the laser beam.

The laser source 110 may include a laser diode 112. The laser diode 112 may be an infrared (IR) laser diode, such as a near-infrared (NIR) laser diode. For example, the laser diode 112 may be configured to generate a raw laser emission 150 in the IR region, such as in the NIR region (750 nm to 6,000 nm), of the electromagnetic spectrum. The raw laser emission 150 may have a wavelength from about 800 nm to about 850 nm. The exact wavelength range of the raw laser emission 150 may depend on manufacturing variation tolerances associated with the laser diode 112.

The raw laser emission 150 may be divergent due to a structure and configuration of the laser diode 112. For example, the laser diode 112 may lack lenses or deflectors to shape and guide the raw laser emission 150. The raw laser emission may include an astigmatism and may lack coherence, as described further with reference to FIG. 1B. The astigmatism may produce an emission that diverges with a small angle (e.g., from 5° to 7°) in one direction and with a larger angle (e.g., from 30° to 40°) in another perpendicular direction.

The raw laser emission 150 may propagate along a laser path 160 that passes through each of the series of lenses 120-140. For example, each of the lenses 120-140 may be aligned along the laser path 160 such that the raw laser emission 150 passes through each of the lenses 120-140.

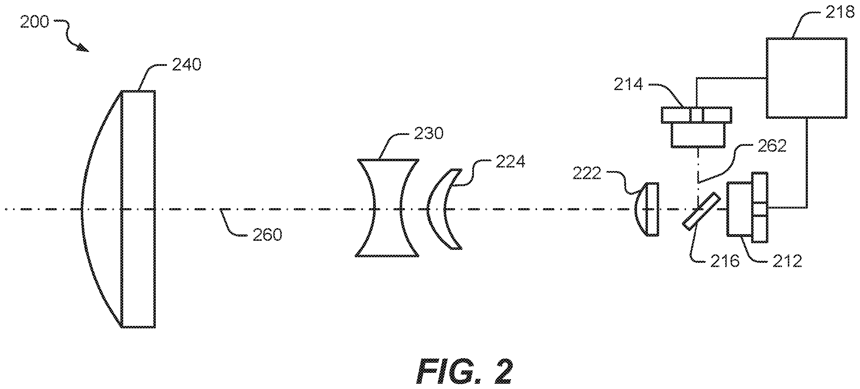

The corrective lens 120 may be positioned in the laser path 160 between the laser diode 112 and the divergence lens 130, and may be configured to correct an astigmatism of the raw laser emission 150 and to collimate the raw laser emission 150, thereby forming a corrected laser emission 152. The corrective lens 120 may further be configured to correct the raw laser emission 150 by making the corrected laser emission 152 circular in cross-section. In order to correct the astigmatism and to perform the collimation, the corrective lens 120 may be a cylindrical or toroidal lens. The corrective lens 120 may further be aspherical, including an aspheric element reducing spherical or optical aberrations, to provide further correction and coherence to the raw laser emission 150. The apparatus 100 may also include a second corrective lens as described with reference to FIG. 2.

The divergence lens 130 may be configured to expand a cross-section of the corrected laser emission 152, thereby forming an expanding laser emission 154. The divergence lens 130 may further be configured to perform additional correction to the corrected laser emission 152. For example, the divergence lens 130 may be aspherical, including an aspheric element reducing spherical or optical aberrations, to adjust the corrected laser emission 152 to increase coherence.

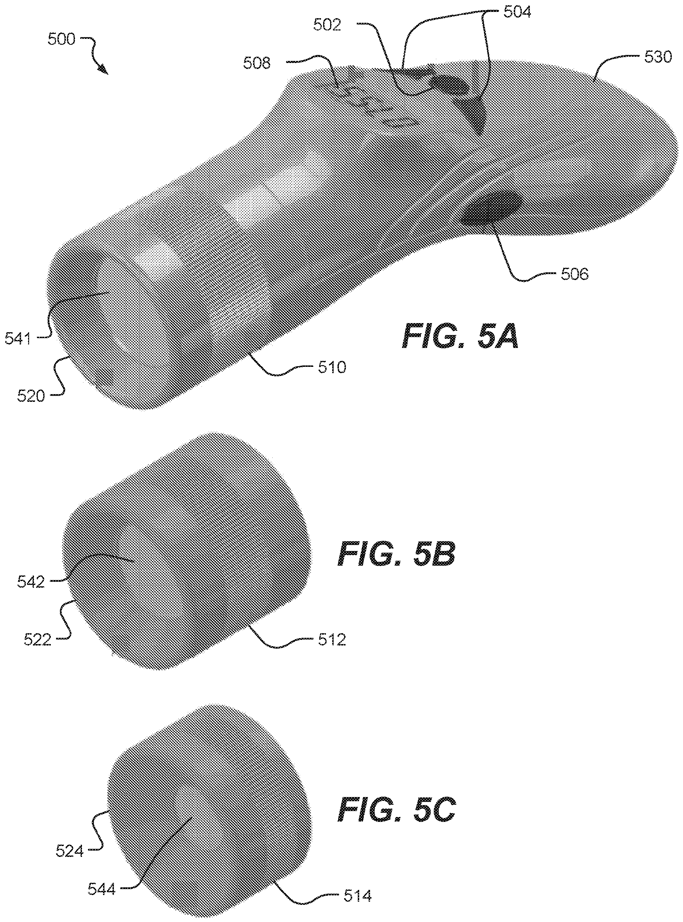

The front lens 140 may be a collimating lens configured to collimate the expanding laser emission 154 to form a high-coherence laser emission 156. The extent to which the raw laser emission 150 is expanded may be based on a distance between the front lens 140 and the divergence lens 130. For example, a larger distance between the front lens 140 and the divergence lens 130 may result in a larger expansion of the raw laser emission 150 and a larger diameter of the high-coherence laser emission 156. Thus, the divergence lens 130 and the front lens 140, together, make a beam expander that increases the beam coherence of the corrected laser emission 152 in proportion to the beam expansion. The distance between the front lens 140 and the divergence lens 130 may be adjustable. As a non-limiting example, the front lens 140 may be included in a front lens assembly that is interchangeable with at least one other front lens assembly including another front lens, as described further with reference to FIGS. 5A-5C. The front lens 140 may be aspherical, including an aspheric element reducing spherical or optical aberrations, to perform additional correction to the expanding laser emission 154, and thereby increase a coherence of the high-coherence laser emission 156.

During operation, the laser diode 112 may generate the raw laser emission 150. The raw laser emission 150 may propagate along the laser path 160 and be received at the corrective lens 120. The corrective lens 120 may correct astigmatism of the raw laser emission 150 and collimate the raw laser emission 150 to form the corrected laser emission 152. The corrected laser emission 152 may be received at the divergence lens 130. The divergence lens 130 may expand a cross-section of the corrected laser emission 152 by forming the expanding laser emission 154. The expanding laser emission 154 may be received at the front lens 140. The front lens 140 may collimate the expanding laser emission 154 to form the high-coherence laser emission 156.

A benefit associated with the apparatus 100 is that an area of the high-coherence laser emission 156 may be larger as compared to low-level laser devices that do not expand laser emissions through a series of lenses such as the lenses 120-140. Due to the expansion, the high-coherence laser emission 156 may have better coherence compared to low-level laser devices that do not include two-stage collimation, such as two-stage collimation with beam expansion between stages. By first correcting and collimating and then diverging and collimating the raw laser emission 150 using the series of lenses 120-140, the apparatus 100 may produce high-coherence laser emissions (e.g., the high-coherence laser emission 156) that are significantly more collimated and coherent than unexpanded laser emissions.

For example, a coherence of the high-coherence laser emission 156 may increase as compared to unexpanded laser emissions by at least the same factor as the expansion. To illustrate, a 5x expansion may produce at least a 5x increase in coherence. The coherence may be further increased through the optical corrections made by the corrective lens 120 and/or by an aspheric shape of any of the series of lenses 120-140.

In applications where the apparatus 100 is used to apply a laser emission to a wound, the increased coherence of the high-coherence laser emission 156 may be more effective in increasing blood flow and/or stimulating healing than devices that do not use lenses to increase coherence. For example, applying the laser emission to a wound may increase vascular flexibility and/or reduce glycohemoglobin in the blood. The laser emission may thus be used to stimulate the healing of wounds, such as in the case of diabetic patients.

Another benefit associated with the apparatus 100 is that by expanding a cross-section of the raw laser emission 150 to produce the high-coherence laser emission 156, the high-coherence laser emission 156 may have a lower peak irradiance as compared to low-level laser devices that do not expand laser emissions after correction and collimation of raw laser emissions. The lower peak irradiance may reduce the risk of injury should the high-coherence laser emission 156 be inadvertently directed into someone's eye.

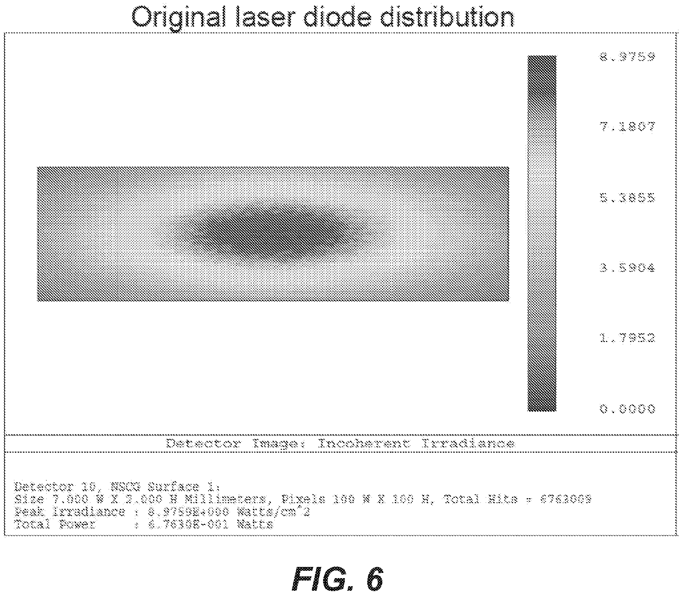

The high-coherence laser emission 156 may also have a more uniform irradiance distribution as compared to unexpanded laser emissions. The more uniform irradiance distribution may enable the laser emission to be applied more evenly over an application area. Specific irradiance distribution benefits are described further with reference to FIGS. 6-9. Other benefits of the apparatus 100 will be apparent to persons of ordinary skill in the art having the benefit of this disclosure.

Referring to FIG. 1B, an illustrated cross-section of the raw laser emission 150 is depicted and generally designated 170. The cross-section 170 may correspond to the indicated cross-section 1B of FIG. 1A and may be perpendicular to the laser path 160. Although FIG. 1B depicts the cross-section 170 as having a defined border, it should be understood that the cross-section 170 represents the shape of the irradiance distribution of the raw laser emission 150 and not a physical border of the raw laser emission 150. For example, the border of the ellipse of FIG. 1B may represent a sharp decline in the irradiance of the raw laser emission 150 as opposed to an absolute cutoff.

As depicted in FIG. 1B, the raw laser emission 150 may be a raw laser emission and may therefore be elliptical in shape. For example, the cross-section 170 may include a minor axis 172 and a major axis 174. Because the raw laser emission 150 expands, the length of the minor axis 172 and the major axis 174 may vary depending on the distance between the cross-section 170 and the laser diode 112. The elliptical shape of the cross-section 170 represents an astigmatism that occurs in the raw laser emission 150 as a result of the structure and generation process used by the laser diode 112.

Referring to FIG. 1C, an illustrated cross-section of the corrected laser emission 152 is depicted and generally designated 180. The cross-section 180 may correspond to the indicated cross-section 1C of FIG. 1A and may be perpendicular to the laser path 160. It should be understood that the cross-section 180 represents the shape of the irradiance distribution of the corrected laser emission 152. A diameter 182 of the cross-section 180 may be based on a shape and size of the corrective lens 120. The diameter 182 may further depend on a distance between the corrective lens 120 and the laser diode 112. A distance between the corrective lens 120 and the laser diode 112 may cause the diameter 182 to be about 8 mm. This may be an approximate value as the exact value of the diameter 182 may further depend on manufacturing variances associated with the corrective lens 120, the laser diode 112, or both.

Referring to FIG. 1D, an illustrated cross-section of the high-coherence laser emission 156 is depicted and generally designated 190. The cross-section 190 may correspond to the indicated cross-section 1D of FIG. 1A and may be perpendicular to the laser path 160. It should be understood that the cross-section 190 represents the shape of the irradiance distribution of the high-coherence laser emission 156. Because the high-coherence laser emission 156 has been enlarged and collimated by the divergence lens 130 and the front lens 140, a diameter 192 of the cross-section may be larger than the diameter 182 of the cross-section 180.

A value of the diameter 192 may depend on a distance between the front lens 140 and the divergence lens 130. For example, a greater distance between the front lens 140 and the divergence lens 130 may result in a greater value of the diameter 192. The diameter 192 may be chosen by selectively replacing the front lens 140 with another interchangeable lens at another distance from the divergence lens 130, as described with reference to FIGS. 5A-5B. To illustrate, the diameter 192 may be chosen by replacing the front lens 140 with a lens such that the cross-section 190 has an area of 2 cm², 4 cm², 6 cm², or another area.

As explained above, by expanding to a larger diameter, the high-coherence laser emission 156 may have better collimation and better coherence as compared to the raw laser emission 150 and/or the corrected laser emission 152. The expanded diameter 192 may further produce a lower peak irradiance and a more uniform irradiance distribution as compared to the cross-section 180 with the diameter 182.

Referring to FIG. 2, a low-level laser therapy apparatus is depicted and generally designated 200. The apparatus 200 includes a first laser diode 212, a second laser diode 214, and a dichroic combiner 216. The apparatus further includes a first corrective lens 222, a second corrective lens 224, a divergence lens 230, and a front lens 240, which together form a series of lenses 222-240 to expand a laser emission from either the first laser diode 212 or the second laser diode 214 to produce a high-coherence laser beam. Similar to the apparatus 100 of FIG. 1, the divergence lens 230 and the front lens 240, together, make a beam expander configured to increase the beam coherence of the corrected laser emission in proportion to the beam expansion. Accordingly, lenses 120-140 may also be used with the first laser diode 212, the second laser diode 214, and the dichroic combiner 216 in FIG. 2.

The first laser diode 212 may be configured to generate a first laser emission along a first laser path 260. The first laser diode 212 may be an IR or NIR laser diode configured to generate the first laser emission in the IR region or NIR region of the electromagnetic spectrum. The first laser emission may have a wavelength from about 800 nm to about 850 nm. The exact wavelength range of the first laser emission may depend on manufacturing variation tolerances associated with the first laser diode 212.

The second laser diode 214 may be configured to generate a second laser emission along a second laser path 262. The wavelength of the second laser emission may be in the visible portion of the electromagnetic spectrum. The second laser emission generated by the second laser diode 214 may have a wavelength from about 480 nm to about 530 nm. For example, the wavelength may correspond to the peak of the sun spectral radiance of approximately 480 nm. Instead, for example, the second laser diode 214 may be a green visible laser diode configured to generate the second laser emission in the green visible region of the electromagnetic spectrum, such as at a wavelength of approximately 525 nm. The exact wavelength may depend on manufacturing variation tolerances associated with the second laser diode 214. By corresponding to the peak of the sun spectral radiance or the green visible region, it is theorized that emissions from the second laser diode 214 may have a healing effect in applications where the apparatus 200 is used to apply a laser emission to a wound.

The dichroic combiner 216 may be configured to combine the first laser path 260 and the second laser path 262 by diverting the second laser path 262. For example, the dichroic combiner 216 may be reflective of the second laser emission generated by the second laser diode 214 and transparent to the first laser emission generated by the first laser diode 121. For example, the dichroic combiner may be reflective or transparent based on a wavelength of a laser emission received at the dichroic combiner. Further, the dichroic combiner 216 may be positioned at a meeting point of the first laser path 260 and the second laser path 262. An angle of the dichroic combiner 216 relative to the first laser path 260 and the second laser path 262 may cause the second laser emission to be reflected, such that the second laser path 262 is diverted along the first laser path 260. The first laser path 260 may remain undisturbed because the first laser emission passes through the dichroic combiner 216 without being reflected. Hence, both the first laser path 260 and the second laser path 262 may be directed through the series of lenses 222-240 along the same path.

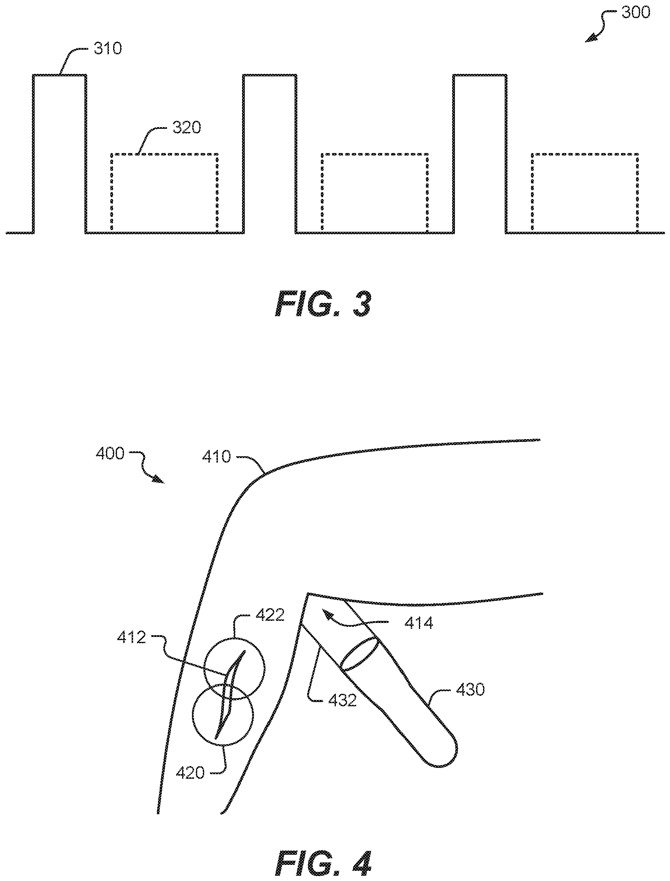

The first laser diode 212 and the second laser diode 214 are controlled by a controller 218. The controller selectively activates the first laser diode 212 and the second laser diode 214. The controller operates the laser diodes 212, 214 in a time-sharing duty cycle such that they are not simultaneously activated and only one of the first laser diode 212 and the second laser diode 214 is directed into the series of lenses 222-240 at a time so that no interference between the radiations occurs. The time-sharing duty cycle is described further with reference to FIG. 3. The controller may include a processor such as a central processing unit (CPU), a digital signal processor (DSP), a peripheral interface controller (PIC), another type of processing element, or a combination thereof. Alternatively or additionally, the controller may include an analog timing device or other circuitry capable of operating the laser diodes 212, 214 in a time-sharing duty cycle as described herein.

The first corrective lens 222 may be positioned in the laser path 260 between the dichroic combiner 216 and the second corrective lens 224, and may be configured to correct an astigmatism of a received laser emission, collimate the received laser emission, or both. In order to correct the astigmatism and/or to perform the collimation, the first corrective lens 222 may be a cylindrical or toroidal lens. The first corrective lens 222 may further be aspherical to provide further correction and coherence to a received laser emission. FIG. 2 shows first corrective lens 222 as a piano convex lens.

The second corrective lens 224 may be positioned in the laser path 260 between the first corrective lens 222 and the divergence lens 230, and may be configured to correct an astigmatism of a received laser emission, collimate the received laser emission, or both. As with the first corrective lens 222, in order to correct the astigmatism and to perform the collimation, the second corrective lens 224 may be a cylindrical or toroidal lens. The second corrective lens 224 may further be aspherical to provide further correction and coherence to a received laser emission. FIG. 2 shows second corrective lens 224 as a positive meniscus lens.

The first corrective lens 222 and the second corrective lens 224 may be configured together to correct an astigmatism of a received laser emission, collimate the received laser emission, or both. For example, the first corrective lens 222 may collimate a received laser emission and the second corrective lens 224 may correct an astigmatism of the received laser emission. Alternatively, the first corrective lens 222 may correct an astigmatism of the received laser emission and the second corrective lens 224 may collimate the received laser emission. The first and second corrective lenses 222, 224 may further be configured to correct a received raw laser emission for making a beam circular in cross-section. For example, the first corrective lens 222, the second corrective lens 224, or both, may be configured to change a shape of a received laser emission, thereby producing a circular beam for expansion. Such a beam may have a diameter of about 8 mm.

The divergence lens 230 may be configured to expand a cross-section of the corrected laser emission received from the second corrective lens 224, thereby forming an expanding laser emission. The divergence lens 230 may further be configured to perform additional correction to the corrected laser emission. For example, the divergence lens 230 may be aspherical to adjust the corrected laser emission to increase coherence. FIG. 2 shows divergence lens 230 as a biconcave lens.

The front lens 240 may be a collimating lens configured to collimate the expanding laser emission to form a high-coherence laser emission. The extent to which a laser emission is expanded may be based on a distance between the front lens 240 and the divergence lens 230. The distance between the front lens 240 and the divergence lens 230 may be adjustable. As a non-limiting example, the front lens 240 may be included in a front lens assembly that is interchangeable with at least one other front lens assembly including another front lens, as described further with reference to FIGS. 5A-5C. The front lens 240 may be aspherical to perform additional correction to the expanding laser emission, and thereby increase a coherence of the high-coherence laser emission. FIG. 2 shows front lens 240 as a piano convex lens.

During operation, a first laser emission may be repeatedly directed from the first laser diode 212 through the series of lenses 222-240 during a first set of time periods. A second laser emission may also be repeatedly directed from the second laser diode 214 through the series of lenses 222-240 during a second set of time periods. The first set of time periods may be distinct from each time period of the second set of time periods. Timing of the first laser emission and the second laser emission may be directed by the controller 218 within a time-sharing duty cycle to reduce or prevent interference between the first laser emission and the second laser emission.

The series of lenses 222-240, configured together, may collimate the received laser emission (e.g., either from the first laser diode 112 or the second laser diode 214) and correct an astigmatism of the received laser emission, thereby forming a corrected laser emission, as described herein. The series of lenses 222-240, when configured together, may also enlarge a cross section of the corrected laser emission, thereby forming an enlarged laser emission, and collimate the enlarged laser emission. In this way, the series of lenses 222-240 may collimate in two stages with expansion between stages and increase coherence of a received laser emission to generate a high-coherence laser emission.

A benefit associated with the apparatus 200 is that the second laser diode 214 may provide a visual indication of an area where an emission from the first laser diode 212 is being applied. Because the first laser diode 212 and the second laser diode 214 are operated at different times, a laser emission from the second laser diode 214 does not interfere with an emission from the first laser diode 212. Further, the visual indication may aid the user in seeing where the laser emission is being applied. Also, because the apparatus 200 gives a visual indication of where an emission of the first laser diode 212 is being applied, a user of the apparatus 200 may avoid inadvertently directing the laser emission in someone's eye.

In cases where the apparatus 200 may be used to stimulate the healing of lesions, another benefit includes the green laser emissions potentially being more effective than other portions of the visible electromagnetic spectrum in stimulating blood flow and/or healing. Wavelengths corresponding to the peak of the sun spectral radiance at around 480 nm may bear similar benefits. Hence, the apparatus 200 may be more effective at stimulating blood flow and/or healing than devices that do not use a visible laser emission.

Another benefit associated with the apparatus 200 is that an area of laser emission produced by the apparatus 200 may be larger as compared to low-level laser devices that do not expand laser emissions through a series of lenses, such as the lenses 222-240. Due to the expansion, the high-coherence laser emission may have better collimation and more coherence. Coherence of the high-coherence laser emission may be further increased through the optical corrections made by the corrective lenses 222, 224 and/or by an aspheric shape of any of the series of lenses 222-240.

By expanding a cross-section of the laser emissions from the laser diodes 212, 214, another benefit associated with the apparatus 200 includes the high-coherence laser emission having a lower peak irradiance as compared to low-level laser devices that do not expand laser emissions. Thereby, apparatus 200 reduces the risk of injury should the high-coherence laser emission be inadvertently directed into someone's eye. The high-coherence laser emission may also have a more uniform irradiance distribution as compared to unexpanded laser emissions, thereby enabling even application over an area. Other benefits of the apparatus 200 will be apparent to persons of ordinary skill in the art having the benefit of this disclosure.

Referring to FIG. 3, a timing diagram illustrating a time-sharing duty cycle for operating the first laser diode 212 and the second laser diode 214 is depicted and generally designated 300. A first function 310 indicates an operating time period corresponding to the first laser diode 212 and a second function 320 indicates an operating time period corresponding to the second laser diode 214. A power output produced by the laser diodes 212, 214 is depicted by the height of the functions 310, 320. For example, the first laser diode 212 may be operated at a higher power and for a shorter duration than the second laser diode 214. However, both may be operated at the same frequency, such as at 25 kHz. The first power output may be from 200 mW to 500 mW, such as 500 mW, and the second power output may be from 100 mW to 300 mW, such as 200 mW. The first laser diode 212 may be operated during 25% to 40%, such as 25%, of the time-sharing duty cycle and the second laser diode 214 may be operated during 25% to 50%, such as 50%, of the time-sharing duty cycle.

Referring to FIG. 4, a diagram of applying a low-level laser with increased coherence to an area is depicted and generally designated 400. The area may be located on a patient's leg 410 and may include a wound 412. A handheld, low-level laser apparatus 430 includes the components of FIG. 2, producing a high coherence laser emission 432. Apparatus 430 may be handheld and thus sized to be capable of operating independent of an external support structure to hold the apparatus during use. Similarly, apparatus 430 may be capable of operating independent of an external power source. Despite the simplicity of its optical design and few optical components shown in FIG. 2, apparatus 430 nonetheless beneficially produces the high coherence laser emission 432 that might previously be produced only by bulkier or more complex devices that are not handheld and/or require an external power source.

Low-level laser apparatus 430 may be used for enhanced wound healing by daily irradiation of each area (e.g., a first area 422 and a second area 420) of the wound 412. Low-level laser apparatus 430 may be used for additional irradiation of the area of blood vessels supplying the wound area (e.g., behind the knee 414). For example, the high-coherence laser emission 432 may be applied to the first area 412 and the second area 420 for eight minutes each day and may be additionally applied behind the knee 414.

By applying the high-coherence laser emission 432 to the wound 412, blood flow to the wound 412 may be increased, thereby increasing a supply of oxygen to the cells in the area of the wound 412. The increase in blood flow may also provide greater capability for immune system cells to reach the wound 412 and increase potential for antibiotics to reach the wound 412.

Referring to FIGS. 5A-5B, a low-level laser apparatus with interchangeable front lens assemblies is depicted and generally designated 500. The apparatus 500 may correspond to the apparatus 100 of FIG. 1, the apparatus 200 of FIG. 2, or another high-coherence low-level laser apparatus. For example, the apparatus 500 may include a base 530 that includes the laser diode 112, the corrective lens 120, and the divergence lens 130, configured as described with reference to FIG. 1. The apparatus 500 may further include a first front lens assembly 520 that includes the front lens 140. As another example, the base 530 may include the first laser diode 212, the second laser diode 214, the dichroic combiner 216, the first corrective lens 222, the second corrective lens 224, and the divergence lens 230, configured as described with reference to FIG. 2. In this example, the front lens assembly 520 may include the front lens 240.

The base 530 may provide a human interface for controlling functions of the apparatus 500. For example, the base 530 may include an on/off button 502 configured to enable selectively applying power to the apparatus 500. To illustrate, the on/off button 502 may be used to toggle a connection between components of the base 530 and a power supply (not shown). The power supply may include a power storage device, such as a battery. The base 530 may further include setup buttons 504. The setup buttons 504 may enable setting a timer for using the apparatus 500. For example, the timer may direct a time period for applying laser emissions from the apparatus 500 to an area. The base 530 may also include a start button 506. The start button may activate a laser emission from the apparatus 500 and start the timer. At the expiration of the timer, the laser emission may be terminated. An amount of time remaining at the timer and a subsequent countdown may be displayed at a display 508. The display 508 may include a liquid crystal diode (LCD) display, a light emitting diode (LED) display, another type of display, or a combination thereof. In addition to the timer, the display 508 may provide an indication of other user settings of the apparatus 500.

As depicted in FIG. 5A, a first front lens assembly 520 may be releasably coupled to the apparatus 500. For example, a bayonet mechanism 510, as is known for camera lenses, may be used to remove the front lens assembly 520 from the base 530 in order to switch to another front lens assembly. The first front lens assembly 520 may include a first front lens 541. The first front lens 541 may be a collimating lens as described with reference to FIGS. 1 and/or 2.

When the first front lens assembly 520 is coupled to the base 530, the first front lens 541 may be positioned in a laser path after a divergence lens to receive a laser emission therethrough after the laser emission passes through the divergence lens. A distance between the first front lens 541 and the divergence lens may be selected such that the apparatus 500 expands a cross-section of a laser emission to have an area of approximately 6 cm² after passing through the first front lens assembly 520.

As depicted in FIG. 5B, a second front lens assembly 522 may be configured to be releasably coupled to the base 530 interchangeably with the first front lens assembly 520. For example, the bayonet mechanism 510 may be used to remove the front lens assembly 520 from the base 530 and a second bayonet mechanism 512 may be used to releasably couple the second front lens assembly 522 to the base 530. The second front lens assembly 522 may include a second front lens 542. The second front lens 542 may also be a collimating lens as described with reference to FIGS. 1 and/or 2.

When the second front lens assembly 522 is coupled to the base 530, the second front lens 542 of the second front lens assembly 522 may be positioned in the laser path after the divergence lens. A distance between the second front lens 542 and the divergence lens when the second front lens is coupled to the base assembly may be different than a distance between the first front lens 541 of the first front lens assembly 520 and the divergence lens. For example, a distance between the second front lens 542 of the second front lens assembly 522 and the divergence lens may be selected such that the apparatus 500 expands a cross-section of a laser emission to have an area of approximately 4 cm² after passing through the second front lens assembly 522.

As depicted in FIG. 5C, a third front lens assembly 524 may be configured to be releasably coupled to the base 530 interchangeably with the first front lens assembly 520 and the second front lens assembly 522. For example, the bayonet mechanism 510 may be used to remove the first front lens assembly 520 from the base 530 or the bayonet mechanism 512 may be used to remove the second front lens assembly 522 from the base 530 and a third bayonet mechanism 514 may be used to releasably couple the third front lens assembly 524 to the base 530. The third front lens assembly 524 may include a third front lens 544. The third front lens 544 may also be a collimating lens as described with reference to FIGS. 1 and/or 2.

When the third front lens assembly 524 is coupled to the base 530, the third front lens 544 of the third front lens assembly 524 may be positioned in the laser path after the divergence lens. A distance between the third front lens 544 and the divergence lens when the third front lens is coupled to the base assembly 530 may be different than a distance between the first front lens 541 of the first front lens assembly 520 and the divergence lens and different than a distance between the second front lens 542 of the second lens assembly 522 and the divergence lens. For example, a distance between the third front lens 544 of the third front lens assembly 524 and the divergence lens may be selected such that the apparatus 500 expands a cross-section of a laser emission to have an area of approximately 2 cm² after passing through the third front lens assembly 524.

A benefit associated with the apparatus 500 is that a user may select an area of a high-coherence laser beam produced by the apparatus 500 dependent on a particular application. For example, a user may change a front lens assembly of the apparatus 500 to select a beam of 2 cm², 4 cm², or 6 cm². Further, when no front lens assembly is attached to the base 530, the resultant laser emission may not be collimated and therefore may continue to expand. As the laser emission expands, peak irradiance of the laser emission may decrease. Thus, peak irradiance of the laser emission may be decreased such that injury may be reduced or prevented if the laser emission is directed in someone's eye while no front lens assembly is coupled to the base 530. Other benefits of the apparatus 500 will be apparent to persons of ordinary skill in the art having the benefit of this disclosure. Although FIGS. 5A-5B depict three (3) front lens assemblies, the apparatus 500 may be configured to receive more or fewer than three (3) front lens assemblies interchangeably.

FIGS. 6-9, are irradiance distribution graphs of hypothetical beams irradiating a surface and showing optical performance for different levels of laser beam expansion. As a validation of the optical design shown in FIG. 2, the software that produced FIGS. 6-9 used one million rays of 808 nm wavelength irradiation originating from a source at varied directions and yielded less than 0.3° of divergence.

Referring to FIG. 6, an irradiance distribution graph of an astigmatic laser emission irradiating a detector is shown. In FIG. 6, the laser emission has an elliptical shape. A major axis of the laser emission is 7 mm and a minor axis of the laser emission is 2 mm. Peak irradiance of the laser emission (depicted at the center of the graph) is approximately 8.9759 W/cm² and edge irradiance (depicted at a corner of the graph) is near 1.7952 W/cm². Total power of the detected radiation is 6.763 x 10⁻¹ W.

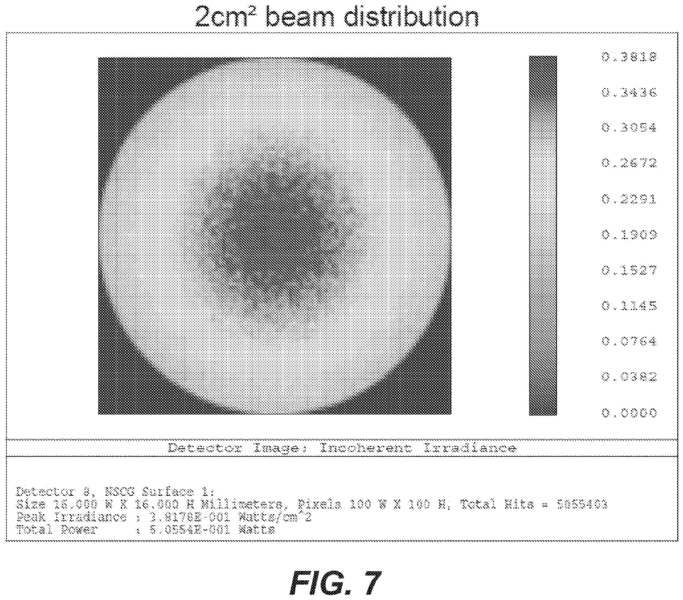

Referring to FIG. 7, an irradiance distribution graph of an expanded laser emission irradiating a detector is shown. The laser emission was modified by a beam expander to have a cross-sectional area of approximately 2 cm². The laser emission of FIG. 7 may correspond to laser emission from the front lens assembly 524 of FIG. 5. As shown in FIG. 7, the laser emission has a circular shape. A diameter of the laser emission is 16 mm. Peak irradiance of the laser emission (depicted at the center of the graph) is approximately 3.8178 x 10⁻¹ W/cm² and an edge irradiance (depicted a corner of the graph) is near zero W/cm². Thus, the irradiance distribution of the FIG. 7 laser emission is more uniform than the laser emission of FIG. 6. Further, peak irradiance of the laser emission of FIG. 7 is lower than peak irradiance of the laser emission of FIG. 6.

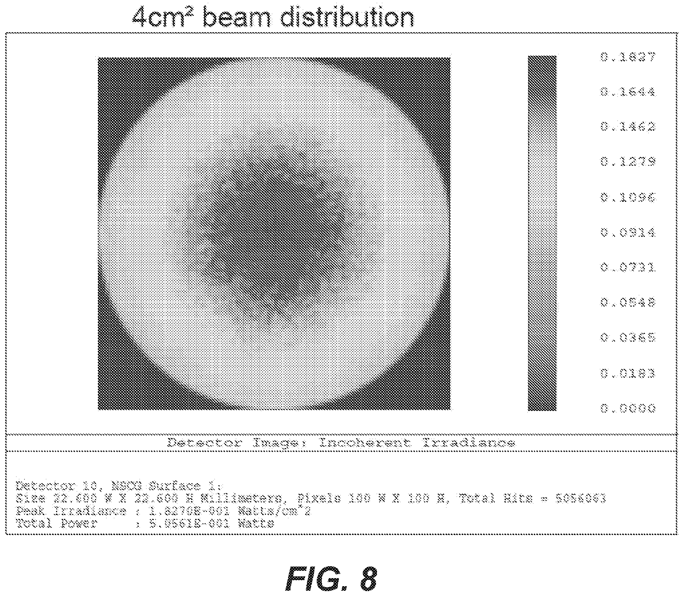

Referring to FIG. 8, an irradiance distribution graph of an expanded laser emission irradiating a detector is shown. The laser emission was modified by a beam expander to have a cross-sectional area of approximately 4 cm². The laser emission of FIG. 8 may correspond to laser emission from the front lens assembly 522 of FIG. 5. As shown in FIG. 8, the laser emission has a circular shape. A diameter of the laser emission is 22.6 mm. Peak irradiance of the laser emission (depicted at the center of the graph) is approximately 1.827 x 10⁻¹ W/cm² and an edge irradiance (depicted at a corner of the graph) is near zero W/cm². Thus, the irradiance distribution of the FIG. 8 laser emission is more uniform than the laser emissions of FIGS. 6 and 7. Further, peak irradiance of the laser emission of FIG. 8 is lower than peak irradiance of the laser emissions of FIGS. 6 and 7.

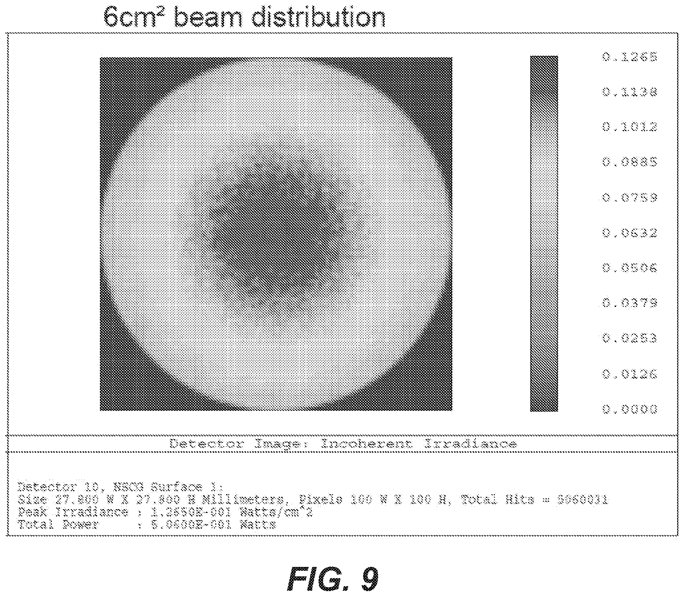

Referring to FIG. 9, an irradiance distribution graph of an expanded laser emission irradiating a detector is shown. The laser emission was modified by a beam expander to have a cross-sectional area of approximately 6 cm². The laser emission of FIG. 9 may correspond to laser emission from the front lens assembly 520 of FIG. 5. As shown in FIG. 9, the laser emission has a circular shape. A diameter of the laser emission is 27.8 mm. Peak irradiance of the laser emission (depicted at the center of the graph) is approximately 1.265 x 10⁻¹ W/cm² and an edge irradiance (depicted at a corner of the graph) is near zero W/cm². Thus, the irradiance distribution of the FIG. 9 laser emission is more uniform than the laser emission of FIGS. 6, 7, and 8. Further, peak irradiance of the laser emission of FIG. 9 is lower than peak irradiance of the laser emission of FIGS. 6, 7, and 8.

Thus, as shown in FIGS. 6-9, as a laser emission is modified by a beam expander, the irradiance distribution of the beam may become more uniform and better defined. Further, peak irradiance of the cross-section is reduced. Thus, the laser emission may be more evenly applied to an area and may present less risk of injury. Referring to the description of FIGS. 5A to 5C, it will be appreciated that the distance between the front lens 541, 542, 544 and the divergence lens may partly control peak irradiance exhibited by the laser emission after passing through the front lens. Other benefits of the apparatuses and methods described herein will be apparent to persons of ordinary skill in the relevant art.

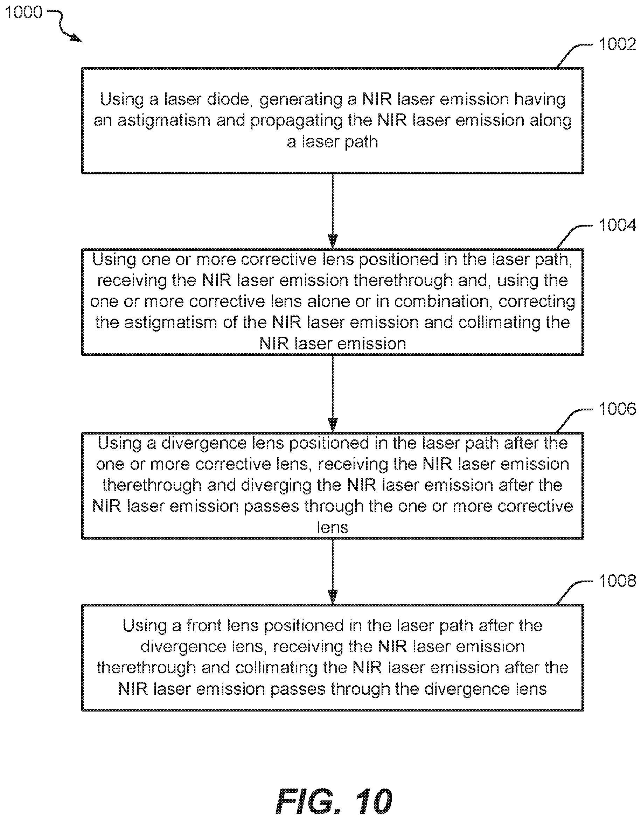

Referring to FIG. 10, a flow chart of a low-level laser beam producing method is depicted and generally designated 1000. The method 1000 may include using a laser diode and generating a laser emission, at 1002. For example, the laser diode 112 may generate the raw laser emission 150.

The method 1000 may also include using a corrective lens positioned in the laser path and receiving the laser emission therethrough, at 1004. For example, the corrective lens 120 may receive the raw laser emission 150 therethrough.

The method 1000 may further include using a divergence lens positioned in the laser path and receiving the laser emission therethrough, at 1006. For example, the corrected laser emission 152 may be received through the divergence lens 130.

The method 1000 may include using a front lens positioned in the laser path after the divergence lens and receiving the laser emission therethrough after the laser emission passes through the divergence lens, at 1008. For example, the diverging laser emission 154 may be received at the front lens 140.

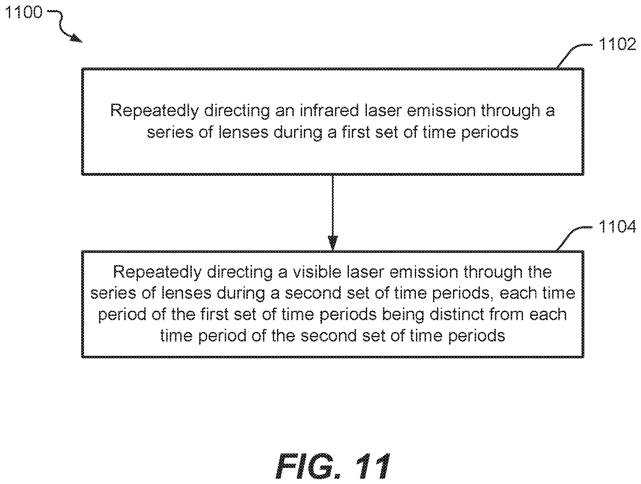

Referring to FIG. 11, a flow chart of a low-level laser beam producing method is depicted and generally designated 1100. The method 1100 may include repeatedly directing an IR laser emission through a series of lenses during a first set of time periods, at 1102. For example, the first laser diode 212 may repeatedly direct an IR laser emission through the lenses 222-240.

The method 1100 may further include repeatedly directing a visible laser emission through the series of lenses during a second set of time periods, at 1104, each time period of the first set of time periods being distinct from each time period of the second set of time periods. For example, the second laser diode 214 may repeatedly direct a visible laser emission through the lenses 222-240.

In the method 1100, the series of lenses may increase a coherence of a received laser emission corresponding to the IR laser emission or the visible laser emission by collimating the received laser emission and correcting an astigmatism of the received laser emission, thereby forming a corrected laser emission. The series of lenses may further enlarge a cross section of the corrected laser emission, thereby forming an enlarged laser emission. The series of lenses may also collimate the enlarged laser emission.

In compliance with the statute, the embodiments have been described in language more or less specific as to structural and methodical features. It is to be understood, however, that the embodiments are not limited to the specific features shown and described. The embodiments are, therefore, claimed in any of their forms or modifications within the proper scope of the appended claims appropriately interpreted.

## Claims

1. A handheld, low-level laser apparatus (100,200,430,500) comprising:
a laser diode (112,212) configured to generate a near infrared (NIR) laser emission having an astigmatism and propagating along a laser path (160,260);
one or more corrective lens (120,222,224) positioned in the laser path (160,260) and configured to receive the NIR laser emission therethrough, the one or more corrective lens (120,222,224) being further configured alone or in combination to correct the astigmatism of the NIR laser emission and to collimate the NIR laser emission;
a divergence lens (130,230) positioned in the laser path (160,260) after the one or more corrective lens (120,222,224) and configured to receive the NIR laser emission therethrough and to diverge the NIR laser emission after the NIR laser emission passes through the one or more corrective lens (120,222,224);
a front lens (140,240,541) positioned in the laser path (160,260) after the divergence lens (130,230) and configured to receive the NIR laser emission therethrough and to collimate the NIR laser emission after the NIR laser emission passes through the divergence lens (130,230);
another laser diode (214) configured to generate a visible laser emission having an astigmatism and propagating along another laser path (262), the one or more corrective lens (120,222,224), the divergence lens (130,230), and the front lens (140,240,541) being configured to receive the visible laser emission therethrough; and
a controller (218) programmed to activate the laser diode (112,212) and the other laser diode (214) in a time-sharing duty cycle such that they are not simultaneously activated.

2. The apparatus (100,200,430,500) of claim 1, wherein the controller (218) is programmed to activate generation of the NIR laser emission during 25% to 40% of the time-sharing duty cycle and wherein the controller (218) is programmed to activate generation of the visible laser emission during 25% to 50% of the time-sharing duty cycle.

3. The apparatus (100,200,430,500) of claim 2, further comprising a dichroic combiner (216) positioned in the laser path (160,260) and in the other laser path, the dichroic combiner (216) being configured to combine the laser path (160,260) and the other laser path (262) into the same path (260) by diverting a propagation direction of either the NIR or visible laser emission.

4. The apparatus (100,200,430,500) of claim 2 or 3, wherein the NIR laser emission has a wavelength from 800 nm to 850 nm and the visible laser emission has a wavelength from 480 nm to 530 nm.

5. The apparatus (100,200,430,500) of any one of claims 2 to 4, wherein the visible laser emission corresponds to the peak of the sun spectral radiance or is in the green visible region of the electromagnetic spectrum.

6. The apparatus (100,200,430,500) of any preceding claim, wherein the one or more corrective lens (120,222,224) is a cylindrical lens or a toroidal lens, or wherein the one or more corrective lens (120,222,224), the divergence lens (130,230), and the front lens (140,240,541) are aspherical.

7. The apparatus (100,200,430,500) of any preceding claim, wherein the one or more corrective lens (120,222,224) is two corrective lenses (222,224) configured together to correct the astigmatism of the laser emission and to collimate the laser emission, preferably wherein the two corrective lenses (222,224) are configured together to yield the laser emission with a substantially circular cross-section, more preferably wherein the circular cross-section has a diameter of approximately 8 mm.

8. The apparatus (100,200,430,500) of any preceding claim, wherein the apparatus (100,200,430,500) is configured such that a distance between the front lens (140,240,541) and the divergence lens (130,230) partly controls peak irradiance exhibited by the laser emission after passing through the front lens (140,240,541).

9. The apparatus (100,200,430,500) of any preceding claim, wherein the laser diode (112,212,214), the one or more corrective lens (120,222,224), and the divergence lens (130,230) are comprised by a base assembly (530) and the front lens (140,240,541) is comprised by a front lens assembly (520) configured to couple releasably to the base assembly (530) to provide the front lens (140,240,541) positioned in the laser path (260),
optionally further comprising a second front lens assembly (522) configured to couple releasably to the base assembly (530) interchangeably with the front lens assembly (520), the second front lens assembly (522) including a second front lens (542) positioned in the laser path (260) after the divergence lens (130,230) when the second front lens assembly (522) is coupled to the base assembly (530), a distance between the second front lens (542) and the divergence lens (130,230) when the second front lens (542) is coupled to the base assembly (530) being different than a distance between the front lens (140,240,541) and the divergence lens (130,230) when the front lens assembly (520) is coupled to the base assembly (530).

10. The apparatus (100,200,430,500) of claim 9, further comprising a third front lens assembly (524) configured to couple releasably to the base assembly (530) interchangeably with the front lens assembly (520) and the second front lens assembly (522), the third front lens assembly (524) including a third front lens (544) positioned in the laser path (260) after the divergence lens (130,230) when the third front lens assembly (524) is coupled to the base assembly (530), a distance between the third front lens (544) and the divergence lens (130,230) when the third front lens (544) is coupled to the base assembly (530) being different than both a distance between the front lens (140,240,541) and the divergence lens (130,230) when the front lens assembly (520) is coupled to the base assembly (530) and a distance between the second front lens (542) and the divergence lens (130,230) when the second front lens assembly (522) is coupled to the base assembly (530).

11. The apparatus (100,200,430,500) of claim 10, wherein a cross-section of the laser emission having an area of approximately 2 cm² after passing through the front lens assembly (520), the cross-section of the laser emission having an area of approximately 4 cm² after passing through the second front lens assembly (522), and the cross-section of the laser emission having an area of approximately 6 cm² after passing through the third front lens assembly (524).

12. A low-level laser beam producing method comprising:
repeatedly directing a near infrared (NIR) laser emission through a series of lenses (120,222,224,130,230,140,240,541,542,544) during a first set of time periods;
repeatedly directing a visible laser emission through the series of lenses (120,222,224,130,230,140,240,541,542,544) during a second set of time periods, each time period of the first set of time periods being distinct from each time period of the second set of time periods, the first set of time periods and the second set of time periods corresponding to a time-sharing duty cycle;
synchronizing the first set of time periods and the second set of time periods using a processor,
the series of lenses (120,222,224,130,230,140,240,541,542,544) increasing the collimation, which increases the coherence, of a received laser emission corresponding to the NIR laser emission, the visible laser emission, or both by:
collimating the received laser emission and correcting an astigmatism of the received laser emission, thereby forming a corrected laser emission;
enlarging a cross section of the corrected laser emission, thereby forming an enlarged laser emission; and
collimating the enlarged laser emission.

13. The method of claim 12, wherein the NIR laser emission has a wavelength from 800 nm to 850 nm and the visible laser emission has a wavelength from 480 nm to 530 nm.

14. The method of claim 12 or 13, wherein repeatedly directing the NIR laser emission through the series of lenses is performed during 25% to 40% of the time-sharing duty cycle, and wherein repeatedly directing the visible laser emission through the series of lenses is performed during 25% to 50% of the time-sharing duty cycle.

15. The method of any one of claims 12 to 14, wherein the visible laser emission corresponds to the peak of the sun spectral radiance or is in the green visible region of the electromagnetic spectrum.

## Patentansprüche

1. Tragbare Low-Level-Laservorrichtung (100, 200, 430, 500), umfassend:
eine Laserdiode (112, 212), welche konfiguriert ist, um eine Nahinfrarot-(NIR)-Laseremission zu erzeugen, welche Astigmatismus aufweist und sich entlang eines Laserpfads (160, 260) ausbreitet;
eine oder mehrere Korrekturlinsen (120, 222, 224), welche in dem Laserpfad (160, 260) angeordnet sind und konfiguriert sind, um die NIR-Laseremission dadurch zu empfangen, wobei die eine oder die mehreren Korrekturlinsen (120, 222, 224) ferner konfiguriert sind, um alleine oder in Kombination den Astigmatismus der NIR-Laseremission zu korrigieren und die NIR-Laseremission zu kollimieren;
eine Divergenzlinse (130, 230), welche in dem Laserpfad (160, 260) nach der einen oder den mehreren Korrekturlinsen (120, 222, 224) angeordnet ist und konfiguriert ist, um die NIR-Laseremission dadurch zu empfangen und die NIR-Laseremission zu divergieren, nachdem die NIR-Laseremission die eine oder die mehreren Korrekturlinsen (120, 222, 224) passiert;
eine Frontlinse (140, 240, 541), welche in dem Laserpfad (160, 260) nach der Divergenzlinse (130, 230) angeordnet ist und konfiguriert ist, um die NIR-Laseremission dadurch zu empfangen und um die NIR-Laseremission zu kollimieren, nachdem die NIR-Laseremission die Divergenzlinse (130, 230) passiert;
eine weitere Laserdiode (214), welche konfiguriert ist, um eine sichtbare Laseremission zu erzeugen, welche Astigmatismus aufweist und sich entlang eines weiteren Laserpfads (262) ausbreitet, wobei die eine oder die mehreren Korrekturlinsen (120, 222, 224), die Divergenzlinse (130, 230) und die Frontlinse (140, 240, 541) konfiguriert sind, um die sichtbare Laseremission dadurch zu empfangen; und
eine Steuerung (218), welche programmiert ist, um die Laserdiode (112, 212) und die weitere Laserdiode (214) in einem Time-Sharing-Arbeitszyklus zu aktivieren, derart, dass sie nicht gleichzeitig aktiviert sind.

2. Vorrichtung (100, 200, 430, 500) nach Anspruch 1, wobei die Steuerung (218) programmiert ist, um die Erzeugung der NIR-Laseremission während 25% bis 40% des Time-Sharing-Arbeitszyklus zu aktivieren und wobei die Steuerung (218) programmiert ist, um die Erzeugung der sichtbaren Laseremission während 25% bis 50% des Time-Sharing-Arbeitszyklus zu aktivieren.

3. Vorrichtung (100, 200, 430, 500) nach Anspruch 2, ferner umfassend einen dichroitischen Kombinator (216), welcher in dem Laserpfad (160, 260) und in dem weiteren Laserpfad angeordnet ist, wobei der dichroitische Kombinator (216) konfiguriert ist, um den Laserpfad (160, 260) und den weiteren Laserpfad (262) in den selben Pfad (260) zu kombinieren, indem eine Ausbreitungsrichtung entweder der NIR-Laseremission oder der sichtbaren Laseremission umgelenkt wird.

4. Vorrichtung (100, 200, 430, 500) nach Anspruch 2 oder 3, wobei die NIR-Laseremission eine Wellenlänge von 800 nm bis 850 nm aufweist und die sichtbare Laseremission eine Wellenlänge von 480 nm bis 530 nm aufweist.

5. Vorrichtung (100, 200, 430, 500) nach einem der Ansprüche 2 bis 4, wobei die sichtbare Laseremission der Spitze der Sonnenspektralstrahlungsdichte entspricht oder in dem grünen sichtbaren Bereich des elektromagnetischen Spektrums liegt.

6. Vorrichtung (100, 200, 430, 500) nach einem der vorhergehenden Ansprüche, wobei die eine oder die mehreren Korrekturlinsen (120, 222, 224) aus einer zylindrischen Linse oder einer ringförmigen Linse bestehen, oder wobei die eine oder die mehreren Korrekturlinsen (120, 222, 224), die Divergenzlinse (130, 230) und die Frontlinse (140, 240, 541) asphärisch sind.

7. Vorrichtung (100, 200, 430, 500) nach einem der vorhergehenden Ansprüche, wobei die eine oder die mehreren Korrekturlinsen (120, 222, 224) aus zwei Korrekturlinsen (222, 224) bestehen, welche gemeinsam konfiguriert sind, um den Astigmatismus der Laseremission zu korrigieren und um die Laseremission zu kollimieren, bevorzugt wobei die zwei Korrekturlinsen (222, 224) gemeinsam konfiguriert sind, um die Laseremission mit einem im Wesentlichen kreisförmigen Querschnitt zu erzeugen, bevorzugter wobei der kreisförmige Querschnitt einen Durchmesser von etwa 8 mm aufweist.

8. Vorrichtung (100, 200, 430, 500) nach einem der vorhergehenden Ansprüche, wobei die Vorrichtung (100, 200, 430, 500) derart konfiguriert ist, dass ein Abstand zwischen der Frontlinse (140, 240, 541) und der Divergenzlinse (130, 230) teilweise die Spitzenstrahlungsdichte steuert, welche die Laseremission aufweist, nachdem diese die Frontlinse (140, 240, 541) passiert hat.

9. Vorrichtung (100, 200, 430, 500) nach einem der vorhergehenden Ansprüche, wobei die Laserdiode (112, 212, 214), die eine oder die mehreren Korrekturlinsen (120, 222, 224) und die Divergenzlinse (130, 230) in einer Basisanordnung (530) enthalten sind und die Frontlinse (140, 240, 541) in einer Frontlinsenanordnung (520) enthalten ist, die konfiguriert ist, um lösbar mit der Basisanordnung (530) gekoppelt zu werden, um die Frontlinse (140, 240, 541) bereitzustellen, die in dem Laserpfad (260) angeordnet ist,
optional ferner umfassend eine zweite Frontlinsenanordnung (522), welche zum lösbaren Koppeln mit der Basisanordnung (530), austauschbar mit der Frontlinsenanordnung (520), konfiguriert ist, wobei die zweite Frontlinsenanordnung (522) eine zweite Frontlinse (542) einschließt, welche in dem Laserpfad (260) nach der Divergenzlinse (130, 230) angeordnet ist, wenn die zweite Frontlinsenanordnung (522) mit der Basisanordnung (530) gekoppelt ist, wobei ein Abstand zwischen der zweiten Frontlinse (542) und der Divergenzlinse (130, 230), wenn die zweite Frontlinse (542) mit der Basisanordnung (530) gekoppelt ist, von einem Abstand zwischen der Frontlinse (140, 240, 541) und der Divergenzlinse (130, 230) unterschiedlich ist, wenn die Frontlinsenanordnung (520) mit der Basisanordnung (530) gekoppelt ist.

10. Vorrichtung (100, 200, 430, 500) nach Anspruch 9, ferner umfassend eine dritte Frontlinsenanordnung (524), welche zum lösbaren Koppeln mit der Basisanordnung (530), austauschbar mit der Frontlinsenanordnung (520) und der zweiten Frontlinsenanordnung (522), konfiguriert ist, wobei die dritte Frontlinsenanordnung (524) eine dritte Frontlinse (544) einschließt, welche in dem Laserpfad (260) nach der Divergenzlinse (130, 230) angeordnet ist, wenn die dritte Frontlinsenanordnung (524) mit der Basisanordnung (530) gekoppelt ist, wobei ein Abstand zwischen der dritten Frontlinse (544) und der Divergenzlinse (130, 230), wenn die dritte Frontlinse (544) mit der Basisanordnung (530) gekoppelt ist, von einem Abstand zwischen der Frontlinse (140, 240, 541) und der Divergenzlinse (130, 230), wenn die Frontlinsenanordnung (520) mit der Basisanordnung (530) gekoppelt ist, und von einem Abstand zwischen der zweiten Frontlinse (542) und der Divergenzlinse (130, 230) unterschiedlich ist, wenn die zweite Frontlinsenanordnung (522) mit der Basisanordnung (530) gekoppelt ist.

11. Vorrichtung (100, 200, 430, 500) nach Anspruch 10, wobei ein Querschnitt der Laseremission eine Fläche von etwa 2 cm² nach dem Passieren der Frontlinsenanordnung (520) aufweist, der Querschnitt der Laseremission eine Fläche von etwa 4 cm² nach dem Passieren der zweiten Frontlinsenanordnung (522) aufweist und der Querschnitt der Laseremission eine Fläche von etwa 6 cm² nach dem Passieren der dritten Frontlinsenanordnung (524) aufweist.

12. Verfahren zum Erzeugen eines Low-Level-Laserstrahls, umfassend:
wiederholtes Richten einer Nahinfrarot-(NIR)-Laseremission durch eine Reihe von Linsen (120, 222, 224, 130, 230, 140, 240, 541, 542, 544) während eines ersten Satzes von Zeitperioden;
wiederholtes Richten einer sichtbaren Laseremission durch die Reihe von Linsen (120, 222, 224, 130, 230, 140, 240, 541, 542, 544) während eines zweiten Satzes von Zeitperioden, wobei jede Zeitperiode des ersten Satzes von Zeitperioden von jeder Zeitperiode des zweiten Satzes von Zeitperioden unterschiedlich ist, wobei der erste Satz von Zeitperioden und der zweite Satz von Zeitperioden einem Time-Sharing-Arbeitszyklus entsprechen;
Synchronisieren des ersten Satzes von Zeitperioden und des zweiten Satzes von Zeitperioden unter Verwendung eines Prozessors,
wobei die Reihe von Linsen (120, 222, 224, 130, 230, 140, 240, 541, 542, 544) die Kollimation erhöhen, welche die Kohärenz einer empfangenen Laseremission erhöht, welche der NIR-Laseremission, der sichtbaren Laseremission, oder beiden entspricht, durch:
Kollimieren der empfangenen Laseremission und Korrigieren eines Astigmatismus der empfangenen Laseremission, wodurch eine korrigierte Laseremission erzeugt wird;
Vergrößern eines Querschnitts der korrigierten Laseremission, wodurch eine vergrößerte Laseremission erzeugt wird; und
Kollimieren der vergrößerten Laseremission.

13. Verfahren nach Anspruch 12, wobei die NIR-Laseremission eine Wellenlänge von 800 nm bis 850 nm aufweist und die sichtbare Laseremission eine Wellenlänge von 480 nm bis 530 nm aufweist.

14. Verfahren nach Anspruch 12 oder 13, wobei das wiederholte Richten der NIR-Laseremission durch die Reihe von Linsen während 25% bis 40% des Time-Sharing-Arbeitszyklus ausgeführt wird, und wobei das wiederholte Richten der sichtbaren Laseremission durch die Reihe von Linsen während 25% bis 50% des Time-Sharing-Arbeitszyklus ausgeführt wird.

15. Verfahren nach einem der Ansprüche 12 bis 14, wobei die sichtbare Laseremission der Spitze der Sonnenspektralstrahlungsdichte entspricht oder in dem grünen sichtbaren Bereich des elektromagnetischen Spektrums liegt.

## Revendications

1. Appareil laser à niveau bas, portatif (100, 200, 430, 500), comprenant :
une diode laser (112, 212) configurée pour générer une émission laser proche-infrarouge (NIR) dotée d'un astigmatisme et se propageant le long d'une trajectoire de laser (160, 260) ;
une ou plusieurs lentilles correctrices (120, 222, 224) positionnées dans la trajectoire de laser (160, 260) et configurées pour recevoir l'émission de laser NIR à travers elles, la ou les lentilles correctrices (120, 222, 224) étant en outre configurées seules ou en combinaison pour corriger l'astigmatisme de l'émission laser NIR et pour collimater l'émission laser NIR ;
une lentille de divergence (130, 230) positionnée dans la trajectoire du laser (160, 260) après l'une ou plusieurs lentilles correctrices (120, 222, 224) et configurée pour recevoir l'émission laser NIR à travers elles et pour dévier l'émission laser NIR après que celle-ci est passée à travers la ou les lentilles correctrices (120, 222, 224) ;
une lentille avant (140, 240, 541) positionnée dans la trajectoire du laser (160, 260) après la lentille de divergence (130, 230) et configurée pour recevoir l'émission laser NIR à travers elle et pour collimater l'émission laser NIR après que l'émission laser NIR passe à travers la lentille de divergence (130, 230) ;
une autre diode laser (214) configurée pour générer une émission laser visible présentant un astigmatisme et se propageant le long d'une autre trajectoire de laser (262), la ou les lentilles correctrices (120, 222, 224), la lentille de divergence (130, 230) et la lentille avant (140, 240, 541) étant configurées pour recevoir l'émission laser visible à travers elles ; et
un système de commande (218) programmé pour activer la diode laser (112, 212) et l'autre diode laser (214) dans un cycle de fonctionnement en temps partagé de sorte qu'elles ne soient pas activées simultanément.

2. Appareil (100, 200, 430, 500) selon la revendication 1, dans lequel le système de commande (218) est programmé pour activer la génération de l'émission laser NIR pendant 25 % à 40 % du cycle de fonctionnement en temps partagé et dans lequel le système de commande (218) est programmé pour activer la génération de l'émission laser visible pendant 25 % à 50 % du cycle de fonctionnement en temps partagé.

3. Appareil (100, 200, 430, 500) selon la revendication 2, comprenant en outre un combineur dichroïque (216) positionné dans la trajectoire du laser (160, 260) et dans l'autre trajectoire de laser, le combineur dichroïque (216) étant configuré pour combiner la trajectoire de laser (160, 260) et l'autre trajectoire de laser (262) dans la même trajectoire (260) en déviant une direction de propagation de l'émission laser NIR ou visible.

4. Appareil (100, 200, 430, 500) selon la revendication 2 ou 3, dans lequel l'émission laser NIR présente une longueur d'onde de 800 nm à 850 nm et l'émission laser visible présente une longueur d'onde de 480 nm à 530 nm.

5. Appareil (100, 200, 430, 500) selon l'une quelconque des revendications 2 à 4, dans lequel l'émission de laser visible correspond au pic de la radiance spectrale solaire ou est dans la région visible verte du spectre électromagnétique.

6. Appareil (100, 200, 430, 500) selon l'une quelconque des revendications précédentes, dans lequel la ou les lentilles correctrices (120, 222, 224) sont une lentille cylindrique ou une lentille toroïdale, ou dans lequel la ou les lentilles correctrices (120, 222, 224), la lentille de divergence (130, 230) et la lentille avant (140, 240, 541) sont asphériques.

7. Appareil (100, 200, 430, 500) selon l'une quelconque des revendications précédentes, dans lequel la ou les lentilles correctrices (120, 222, 224) sont deux lentilles correctrices (222, 224) configurées ensemble pour corriger l'astigmatisme de l'émission laser et pour collimater l'émission laser, de préférence dans lequel les deux lentilles correctrices (222, 224) sont configurées ensemble pour donner l'émission laser avec une section transversale sensiblement circulaire, de préférence dans lequel la section transversale circulaire présente un diamètre d'approximativement 8 mm.

8. Appareil (100, 200, 430, 500) selon l'une quelconque des revendications précédentes, dans lequel l'appareil (100, 200, 430, 500) est configuré de sorte qu'une distance entre la lentille avant (140, 240, 541) et la lentille de divergence (130, 230) contrôle partiellement le pic d'irradiance présenté par l'émission laser après son passage à travers la lentille avant (140, 240, 541).

9. Appareil (100, 200, 430, 500) selon l'une quelconque des revendications précédentes, dans lequel la diode laser (112, 212, 214), la ou les lentilles correctrices (120, 222, 224), et la lentille de divergence (130, 230) sont compris dans un ensemble de base (530) et la lentille avant (140, 240, 541) est comprise dans un ensemble de lentille avant (520) configuré pour se raccorder de manière détachable à l'ensemble de base (530) pour fournir la lentille avant (140, 240, 541) positionnée dans la trajectoire de laser (260),
optionnellement comprenant en outre un deuxième ensemble de lentille avant (522) configuré pour se raccorder de manière détachable à l'ensemble de base (530) de manière interchangeable avec l'ensemble de lentille avant (520), le deuxième ensemble de lentille avant (522) incluant une deuxième lentille avant (542) positionnée dans la trajectoire du laser (260) après la lentille de divergence (130, 230), quand le deuxième ensemble de lentille avant (522) est raccordé à l'ensemble de base (530), une distance entre la deuxième lentille avant (542) et la lentille de divergence (130, 230) quand la deuxième lentille avant (542) est raccordée à l'ensemble de base (530) étant différente d'une distance entre la lentille avant (140, 240, 541) et la lentille de divergence (130, 230) quand l'ensemble de lentille avant (520) est raccordée à l'ensemble de base (530).

10. Appareil (100, 200, 430, 500) selon la revendication 9, comprenant en outre un troisième ensemble de lentille (524) configuré pour se raccorder de manière détachable à l'ensemble de base (530) de manière interchangeable avec l'ensemble de lentille avant (520) et le deuxième ensemble de lentille avant (522), le troisième ensemble de lentille avant (524) incluant une troisième lentille avant (544) positionnée dans la trajectoire du laser (260) après la lentille de divergence (130, 230) quand le troisième ensemble de lentille avant (524) est raccordé à l'ensemble de base (530), une distance entre la troisième lentille avant (544) et la lentille de divergence (130, 230) quand la troisième lentille avant (544) est raccordée à l'ensemble de base (530) étant différente d'une distance entre la lentille avant (140, 240, 541) et la lentille de divergence (130, 230) quand l'ensemble de lentille avant (520) est raccordé à l'ensemble de base (530) et une distance entre la deuxième lentille avant (542) et la lentille de divergence (130, 230) quand le deuxième ensemble de lentille avant (522) est raccordé à l'ensemble de base (530).

11. Appareil (100, 200, 430, 500) selon la revendication 10, dans lequel une section transversale de l'émission de laser présente une superficie d'approximativement 2 cm² après son passage à travers l'ensemble de lentille avant (520), la section transversale de l'émission laser présente une superficie d'environ 4 cm² après son passage à travers le deuxième ensemble de lentille avant (522), et la section transversale de l'émission laser présente une superficie d'approximativement 6 cm² après son passage à travers le troisième ensemble de lentille avant (524).

12. Procédé de production d'un faisceau laser à bas niveau comprenant :
la direction répétée d'une émission laser proche infrarouge (NIR) à travers une série de lentilles (120, 222, 224, 130, 230, 140, 240, 541, 542, 544) pendant un premier ensemble de laps de temps ;
la direction répétée d'une émission laser visible à travers la série de lentilles (120, 222, 224, 130, 230, 140, 240, 541, 542, 544) pendant un second ensemble de laps de temps, chaque laps de temps du premier ensemble de laps de temps étant distinct de chaque laps de temps du second ensemble de laps de temps, le premier ensemble de laps de temps et le second ensemble de laps de temps correspondant à un cycle de fonctionnement en temps partagé ;
la synchronisation du premier ensemble de laps de temps et du second ensemble de laps de temps en utilisant un processeur,
la série de lentilles (120, 222, 224, 130, 230, 140, 240, 541, 542, 544) augmentant la collimation, qui augmente la cohérence, d'une émission laser reçue correspondant à l'émission laser NIR, l'émission laser visible ou les deux en :
collimatant l'émission laser reçue et corrigeant un astigmatisme de l'émission laser reçue, en formant ainsi une émission laser corrigée ;
élargissant une section transversale de l'émission laser corrigée, en formant ainsi une émission laser élargie ; et
collimatant l'émission laser agrandie.

13. Procédé selon la revendication 12, dans lequel l'émission laser NIR présente une longueur d'onde de 800 nm à 850 nm et l'émission laser visible présente une longueur d'onde de 480 nm à 530 nm.

14. Procédé selon la revendication 12 ou 13, dans lequel la direction répétée de l'émission laser NIR à travers la série de lentilles est réalisée pendant 25 % à 40 % du cycle de fonctionnement en temps partagé et dans lequel la direction répétée de l'émission laser visible à travers la série de lentilles est réalisée pendant 25 % à 50 % du cycle de fonctionnement en temps partagé.

15. Procédé selon l'une quelconque des revendications 12 à 14, dans lequel l'émission laser visible correspond au pic de la radiance spectrale solaire ou est dans la région visible verte du spectre électromagnétique.
